# EUROPEAN PATENT APPLICATION

(11) **EP 1 652 499 A2**
(43) Date of publication of application: **03.05.2006**
(21) Application number: 05466016.2
(22) Date of filing: 25.10.2005
(51) Int. Cl.: A61F 13/00, A61F 13/12, A41D 1/00

(54) **A bandage material with active carbon fibres**

(30) Priority: 01.11.2004 CZ 20041086
(71) Applicant: Bauer, Vaclav, 263 01 Dobris (CZ)
(72) Inventor: Bauer, Vaclav, 263 01 Dobris (CZ)
(74) Representative: Loskotova, Jarmila

(57) **Abstract**

The bandage material with active carbon fibres, which consists of one load-bearing layer /1/ made of unwowen viscose/polypropylene textile, and a second active layer /2/ made of 200g/m² pure carbon fibre; both layers form a compact union. The bandage material to be used on bleeding and other smaller surface wounds, etc., consists of a load-bearing layer /3/, which can have any flat shape, and its part houses bandage material with active carbon fibres, oriented with active carbon fibre layer on the top /2/, with the edges of the load-bearing layer fitted with adhesive layer with peel-away protective cover /5/.

## Description

### The technical field

The invention concerns health-care bandage material with active carbon fibres for dressing of wounds.

### The prior art

Many types of bandage materials are use to dress wounds in the health-care sector, either dry materials used only to cover the wound, or materials impregnated with various salves or other means of increasing the speed of closing of the wound. However, these materials are inappropriate for purulent wounds, because they cling to the wound and when replacement is necessary, old bandage must be tom away; this slows down the healing.

For purulent and other wounds which heal slowly, a bandage material containing active carbon is more appropriate for its adsorbent qualities.

Vliwaktiv is one of examples of multilayer bandage materials present at the market. It consists of an outer layer which protects the clothes, a cellulose layer, a cloth made of viscose with active carbon, and a special layer applied directly to the wound.

It is known that active carbon absorbs smells, neutralizes unpleasing odors of infected and septic wounds. Active carbon also can neutralize bacteria.

However, active adsorption layer of this bandage material, i.e. compressed carbon particles, are not in direct contact with the wound area, and thus is not active. The layer in direct contact with the wound clings to it and thus slows down the healing.

Another commercially available material, ACTISORB PLUS, has layered structure similar to Vliwaktiv with active carbon, enriched with silver. This bandage material adsorbs the seaetion into cellulose layer, and the silver contained in active carbon layer destroys germs. In this case, the carbon layer is also on a substrate. Carbon layer is not in direct contact with the wound, which once again reduces the effectiveness of this bandage material, and the bandage clings to the wound.

All the above mentioned bandage materials have their active carbon layer or carbon fibres encapsulated, which reduces their effectiveness.

All the above mentioned bandage materials are thick and hard to manipulate with.

Until now, carbon fibres, wowen or unwowen, have been used as the active layer, but always encapsulated. Until now, no bandage material had active carbon or carbon fibre layer applied directly to the wound surface.

### The nature of the invention

The above mentioned disadvantages can be greatly reduced by the proposed bandage material with active carbon fibres, which consists of one load-bearing layer made of unwowen viscose/polypropylene textile, and a second layer made of wowen or unwowen carbon fibre layer, both layers form a compact union.

The layers are bonded by a heat-activated process, e.g. welding, fusion, etc., using a non-toxic thermally-reactive powder.

Load-bearing layer made of viscose and polypropylene is produced by a special machine using viscose: polypropylene 1:1.

Load-bearing layer can also be made of polypropylene, viscose cuts or fibres, or a part of viscose can be injected between the polypropylene and viscose layers at higher temperature, thus bonding them.

Active layer can cover the whole load-bearing layer, or can only cover parts of the load-bearing layer.

Bandage material can consist of several layers; various combinations of load-bearing and active layers are possible.

According to the invention, an advantageous solution is to use a layer of material with carbon fibres on top of a substrate, which extends over the edges of the active layer and adhesive layer is applied to these non-active edges. Adhesive layer has protective coating, which is peeled away before use, active layer is attached to wounded area and adhesive perimeter attaches to surrounding healthy skin. This type of bandage material with active carbon fibres is used to dress bleeding wounds and also smaller deep wounds. Bandage material with active carbon fibres is breathable and if used with a non-breathable substrate, any shape can be formed. It poses no problem when the bandage material is for example rectangular, and the adhesive layer is at all sides.

A disinfecting face mask protecting the mouth and nose is yet another possible application form of this active carbon fibre material. The bandage material described above is shaped to cover the mouth and nose and consists of at least one layer of carbon fibre bandage material according to the invention, and of protective layers at both sides. The side with active carbon fibres is applied to the mouth and nose. The face mask also has normal fittings which attach it to the ears or the head. The face mask can also be manufactured solely of the active carbon fibre layer and protective layers at both sides.

### Effects of the invention.

Bandage material according to the invention has led to creation of safe load-bearing layer, which hosts an active layer made of carbon fibres, where the resulting compact unit allows the active layer to be laid directly onto the wound, as it does not separate nor break. This allows the good properties of carbon fibres - high sorption speed and capacity for germs, toxins, chemical and toxic substances and odors - to be used.

The wound, dressed with the material according to the invention, clears up and heals very fast; the invention facilitates re-granulation.

Creation of a suitable load-bearing layer is a result of long-term clinical trials and tests, which are documented.

Active layer made of carbon fibres can be produced in different thicknesses. It can be very thin, if required; this is advantageous. Active layer made of carbon fibres maintains its good qualities even when thin.

The weave made of carbon fibres is non-toxic, does not provoke adverse reactions, it is breathable, hydrophilic, but with low water adsorption.

Carbon weave contains no foreign substances, which could irritate or reduce adsorption.

The design and mechanical properties of bandage material according to the invention allow direct contact of carbon fibres with wound surface, where they effect their adsorption capacity.

Active layer is a weave made of hydrated cellulose fibre, subjected to controlled carbonization and activation at temperatures of several hundreds °C. This thermic treatment results in pure activated carbon shaped as original weave, with microporous structure of fibres, resulting in high adsorption of organic matters.

Carbon is extremely chemically stable and heat resistant; it is resistant against hydrolysis, photolysis, and also against oxidation and thermal decomposition at normal temperatures. It needs high temperatures to combine with oxygen, sulphur, and other halides. It does not dissolve in any solvents, it dissolves in molten metals. This guarantees exceptional physically chemical stability of the active layer.

The bandage material according to the invention is not intended for absorption of secretion.

It has only small absorption capacity and therefore it is not intended for absorption of secrets, which are to be absorbed into other absorptive material, which is applied over the invention.

Active layer does not cling to the wound; it can be left in place for longer period, because it heals and disinfects the wound and adsorbs odors. Only the top absorptive material is normally replaced.

The bandage material according to the invention is used in health care for treatment of various wounds, trophic, purulent, complicated post-surgery wounds with bacterial contamination, etc., and it is also used to eliminate unpleasant odors.
- active layer can be applied directly onto the wound, thus the effects are better than in currently known bandages, even those including carbon fibres
- easy application
- has anti-odour properties
- actively traps germs
- actively traps chemical agents
- prevents secondary infections
- has anti-odour properties
- speeds up healing of treated surfaces
- is non-toxic
- is not aggressive
- does not lead to adverse effects when applied to a location
- adapts well to the surface of the defect
- speeds up healing of treated surfaces
- easy and simple application
- patients tolerate this bandage material well
- is sanitized and packaged according to healthcare standards

Face masks made of material with active carbon fibres can be used, for above mentioned reasons, in chemically contaminated areas or operations instead of masks, provided that leaks around nose and mouth are prevented. Face masks according to the invention are very suitable for wearing within areas harmful to health.

### List of pictures in the drawina

Bandage material with active carbon fibres is described in more detail at appended drawings, where: Fig. 1 shows the bandage material according to the invention in basic version, Fig. 2 shows the bandage material with active carbon fibres in rectangular shape with adhesive perimeter, Fig. 3 shows the bandage material with active carbon fibre as a face mask, where fig. a) is the front view, fig. b) is a cross section showing how the face mask is formed, and fig. c) is a cross section without folds.

### Sample realization of the invention

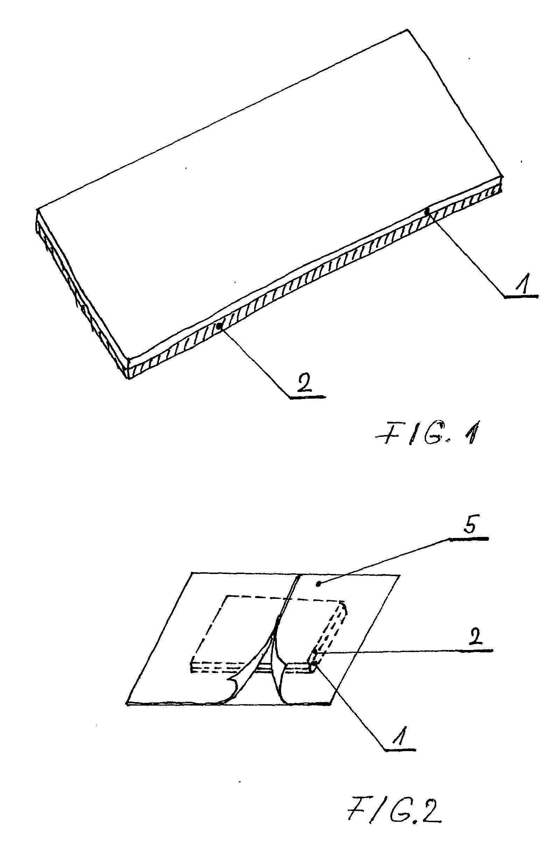

The bandage material according to fig. 1 consists of one inactive white load-bearing layer 1 made of non-wowen textile, including one layer of 38 % of viscose cuts and another layer of 50 % of polypropylene cuts, and of 12 % of liquid viscose, which is sprayed between the two layers during the production, for total weight 120g/m²; this load-bearing layer 1 is a base for a second, active, black layer 2 made of 200g/m² pure carbon fibre, attached using non-toxic thermally activated powder, both layers form a compact unit.

Active layer 2 of the bandage material is applied directly to the wound. Handling of this bandage is simple and the patient can handle it alone. The bandage material is highly hydrophilic, breathable, permeable, and very suitable for adsorption of germs and organic matter. It has only small absorption capacity and is therefore not intended for absorption of secrets, which have to be absorbed by other absorbent material, covering the bandage material according to the invention.

Active layer 2 contains no organic fraction of other impurities subject to decay. It consists of virtually pure carbon with < 1 % remains after annealing.

The bandage material with active carbon fibres depicted in fig. 2 consists of a substrate 3, whose part indudes the bandage material with carbon fibres, consisting of inactive load-bearing layer 1, active carbon fibres layer 2, with adhesive edges 4 of the substrate 3 on the side with active carbon fibres; adhesive edges are covered with protective coat 5, which at the same time protects the active carbon fibre layer 2. When the protective coat 5 is peeled away, the bandage material with active carbon fibres is pressed onto the wound by its active side 2, and the adhesive edges are pressed onto surrounding healthy skin.

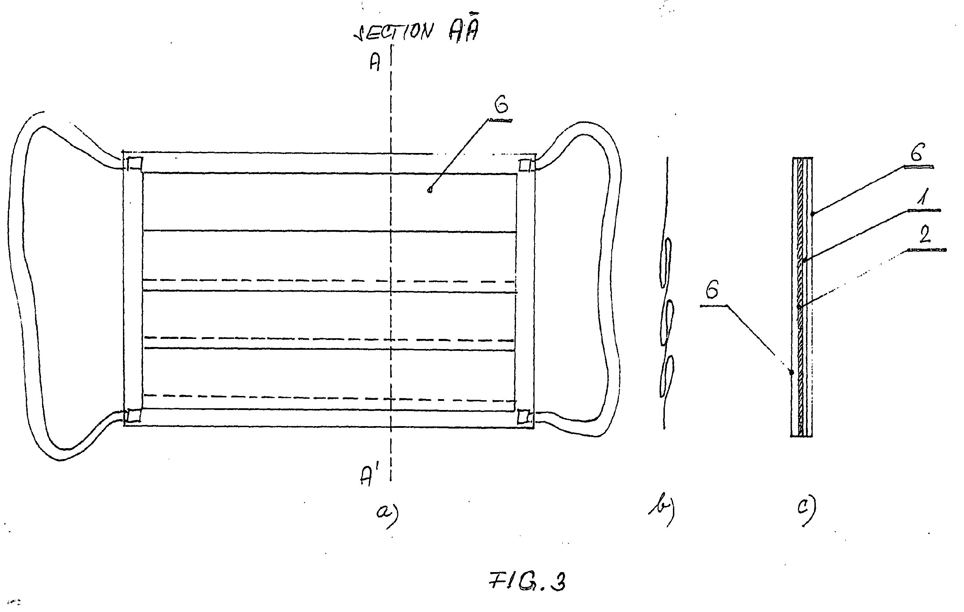

The bandage material with active carbon fibres depicted in fig. 3 has rectangular shape and consists of three layers, of which the middle one is the bandage material with active carbon fibres consisting of inactive layer 1, active carbon fibre layer 2, and protected from both sides with protective layer 6; the whole face mask has fittings which allow fastening to the ears or the head. The active carbon fibre surface of the face mask is applied to the mouth and nose and only lightly covered by one of protective layers 6. The face mask can also be manufactured in a way where all layers are doubled over longitudinally, reinforced at the sides, and opening in the middle to allow insertion of mouth and nose.

### Possible uses in the industry

The bandage material according to the invention has broad application possibilities in the health care. It can be used in dermatology for shank sores, sores typical for diabetes mellitus, hard-to-heal wounds with secondary infection, open infected tumor defects, bums, post-surgery wounds, etc. Bandage material with adhesive edges is more suitable for small bleeding wounds. Bandage material with active carbon fibres shaped as face mask is suitable to the locations where face masks are worn, but it has much better effectiveness. Therefore, face masks according to the invention are suitable also for the environments where masks should be used.

## Claims

1. A bandage material with active carbon fibres characteristic in that it consists of one load-bearing layer /1/ made of non-wowen textile, consisting of viscose and polypropylene, and of a second active layer /2/, consisting of pure carbon fibre; both layers form a compact unit.

2. A bandage material with active carbon fibres, according to daim 1., chara cteristic in that both layers are bonded by a heat-activated process, e.g. welding, fusion, etc., using a non-toxic thermally-reactive powder.

3. A bandage material with active carbon fibres, according to claim 1., chara cteristic in that the active layer /2/ covers the whole inactive load-bearing area /1/ or part thereof.

4. A bandage material with active carbon fibres, according to claim 1., chara cteristic in that it consists of several layers with different possible combinations of load-bearing layer /1/ and active layer /2/.

5. A bandage material with active carbon fibres, according to claim 1., chara cteristic in that it consists of a load-bearing layer /3/, which can have any flat shape, and its part houses bandage material with active carbon fibres, oriented with active carbon fibre layer /2/ on the top, with the edges /4/ of the load-bearing layer /3/ which are fitted with adhesive layer with protective cover /5/, which at the same time also protects the active carbon fibre layer /2/.

6. A bandage material with active carbon fibres, according to claim 1., shaped as face mask, with fittings to allow attachment to the ears or the head, chara cteristic in that the face mask consists of at least one layer of bandage material with active carbon fibres, or of an active carbon fibre layer covered by protective layer /6/ on both sides, forming one unit.

7. A bandage material with active carbon fibres, according to daim 1., chara cteristic in that all layers forming the face mask are doubled over longitudinally, reinforced at the sides, and opening in the middle to allow insertion of mouth and nose.
